# EUROPEAN PATENT SPECIFICATION

(11) **EP 4 104 763 B1**
(45) Date of publication and mention of the grant of the patent: **31.07.2024**
(21) Application number: 22177786.5
(22) Date of filing: 08.06.2022
(51) Int. Cl.: A61B 5/367, A61B 5/287, A61B 5/00

(54) **WAVE PROPAGATION CONTROL ENHANCEMENT**
VERBESSERUNG DER WELLENAUSBREITUNGSSTEUERUNG
AMÉLIORATION DU CONTRÔLE DE LA PROPAGATION D'ONDES

(30) Priority: 09.06.2021 US 202117342871
(43) Date of publication of application: 21.12.2022
(73) Proprietor: Biosense Webster (Israel) Ltd., Yokneam 2066717 (IL)
(72) Inventor: ALTMAN, Sigal, 2066717 Yokneam (IL); MASSARWA, Fady, 2066717 Yokneam (IL); ZOHAR, Gal Bar, 2066717 Yokneam (IL)
(74) Representative: Carpmaels & Ransford LLP

(56) References cited:
- US-A1- 2018 256 056
- US-A1- 2019 076 045
- US-A1- 2020 113 465
- US-A1- 2020 297 234
- US-A1- 2021 106 243

## Description

### FIELD OF THE INVENTION

The present invention relates to medical systems, and in particular, but not exclusively to, catheter-based systems.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied to the tip electrode(s) of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Therefore, when placing an ablation or other catheter within the body, particularly near the endocardial tissue, it is desirable to have the distal tip of the catheter in direct contact with the tissue. The contact can be verified, for example, by measuring the contact between the distal tip and the body tissue. U.S. Patent Application Publication Nos. 2007/0100332, 2009/0093806 and 2009/0138007, describe methods of sensing contact pressure between the distal tip of a catheter and tissue in a body cavity using a force sensor embedded in the catheter.
US 2021/106243 A1 discusses methods, apparatus, and systems for medical procedures which include receiving a first set of biometric data for a first portion of a body part and determining a first range of values in the first set of biometric data, determining first visual characteristics based on the first range of values and rendering a global view of the first portion of the body part rendered with the first visual characteristics. A second range of values in a second set of biometric data for a second portion of the body part may be determined and the second portion of the body part may be a subset of the first portion of the body part. Second visual characteristics may be determined based on the second range of values and a local view including the second portion of the body part rendered with the second visual characteristics may be rendered. For example, the global view may show a cardiac chamber with LAT values that range from -500 ms through +500 ms indicated by colors and the local view, superimposed onto a portion of the global view over a smaller portion of the cardiac chamber may show LAT values that range from -200 through +150 ms.

### SUMMARY

There is provided in accordance with an embodiment of the present disclosure, a medical system for wave propagation enhancement, the medical system comprising: a catheter configured to be inserted into a chamber of a heart, and including electrodes configured to capture electrical activity of tissue of the chamber over time;a display; and processing circuitry configured to:identify local activation times (LATs) from the captured electrical activity and associate the local activation times with respective positions on the surface of an anatomical map of the chamber;compute a propagation of a cardiac activation wave over the anatomical map of the chamber from a start time in a cardiac cycle to an end time in the cardiac cycle responsively to the captured electrical activity, wherein the propagation of the cardiac activation wave is calculated using a sliding window of the local activation times;render to the display a sub-region of the anatomical map;select a time-bounded portion of the propagation of the cardiac activation wave commencing at a time after the start time, wherein the time after the start time is the earliest time the wave would arrive in the sub-region of the anatomical map; and render to the display the time-bounded portion of the propagation of the cardiac activation wave on the sub-region of the anatomical map.

Further in accordance with an embodiment of the present disclosure the processing circuitry is configured to select the time-bounded portion of the propagation of the cardiac activation wave ending at a time before the end time responsively to when the propagation of the cardiac activation wave would complete to be rendered in the sub-region of the anatomical map.

Still further in accordance with an embodiment of the present disclosure the processing circuitry is configured to automatically repeat rendering of the time-bound portion of the propagation of the cardiac activation wave on the sub-region of the anatomical map.

Additionally, in accordance with an embodiment of the present disclosure the processing circuitry is configured to render the sub-region of the anatomical map from a viewpoint within the anatomical map.

Moreover, in accordance with an embodiment of the present disclosure the processing circuitry is configured to render the sub-region of the anatomical map from a viewpoint within the anatomical map, while the viewpoint is static during rendering of the time-bound portion of the propagation of the cardiac activation wave on the sub-region of the anatomical map.

Further in accordance with an embodiment of the present disclosure, the system includes a user interface to receive user input of a manipulation of a virtual camera to change a rendered view of the anatomical map from within the anatomical map, wherein the processing circuitry is configured to render the sub-region of the anatomical map responsively to the user input.

Still further in accordance with an embodiment of the present disclosure the processing circuitry is configured to render the sub-region of the anatomical map from a viewpoint outside of the anatomical map.

Additionally, in accordance with an embodiment of the present disclosure, the system includes a user interface to receive user input of a selection of the sub-region of the anatomical map, wherein the processing circuitry is configured to render the sub-region of the anatomical map responsively to the user input.

Moreover, in accordance with an embodiment of the present disclosure, the system includes a user interface to receive user input of a speed of the rendering of the time-bounded portion of the propagation of the cardiac activation wave, wherein the processing circuitry is configured to render to the display the time-bound portion of the propagation of the cardiac activation wave on the sub-region of the anatomical map responsively to the user input of the speed.

Further in accordance with an embodiment of the present disclosure, the system includes a user interface to receive user input of a width of the cardiac activation wave, wherein the processing circuitry is configured to render to the display the time-bound portion of the propagation of the cardiac activation wave on the sub-region of the anatomical map responsively to the user input of the width of the cardiac activation wave.

There is also provided in accordance with another embodiment of the present disclosure, a medical method for wave propagation control enhancement, the method comprising: identifying local activation times (LATs) from electrical activity of tissue of a chamber of a heart captured by electrodes of a catheter inserted into the chamber and associating the local activation times with respective positions on the surface of an anatomical map of the chamber of the heart;computing a propagation of a cardiac activation wave over the anatomical map of the chamber of the heart from a start time in a cardiac cycle to an end time in the cardiac cycle responsively to the captured electrical activity, wherein the propagation of the cardiac activation wave is calculated using a sliding window of the local activation times rendering a sub-region of the anatomical map to a display; selecting a time-bounded portion of the propagation of the cardiac activation wave commencing at a time after the start time, wherein the time after the start time is the earliest time the wave would arrive in the sub-region of the anatomical map; and rendering the time-bounded portion of the propagation of the cardiac activation wave on the sub-region of the anatomical map to the display.

Still further in accordance with an embodiment of the present disclosure the selecting includes selecting the time-bounded portion of the propagation of the cardiac activation wave ending at a time before the end time responsively to when the propagation of the cardiac activation wave would complete to be rendered in the sub-region of the anatomical map.

Additionally, in accordance with an embodiment of the present disclosure, the method includes automatically repeating rendering of the time-bound portion of the propagation of the cardiac activation wave on the sub-region of the anatomical map.

Moreover, in accordance with an embodiment of the present disclosure the rendering the sub-region includes rendering the sub-region of the anatomical map from a viewpoint within the anatomical map.

Further in accordance with an embodiment of the present disclosure the rendering the sub-region includes rendering the sub-region of the anatomical map from a viewpoint within the anatomical map, while the viewpoint is static during rendering of the time-bound portion of the propagation of the cardiac activation wave on the sub-region of the anatomical map.

Still further in accordance with an embodiment of the present disclosure, the method includes receiving user input of a manipulation of a virtual camera to change a rendered view of the anatomical map from within the anatomical map, wherein the rendering the sub-region includes rendering the sub-region of the anatomical map responsively to the user input.

Additionally, in accordance with an embodiment of the present disclosure the rendering the sub-region includes rendering the sub-region of the anatomical map from a viewpoint outside of the anatomical map.

Moreover, in accordance with an embodiment of the present disclosure, the method includes receiving user input of a selection of the sub-region of the anatomical map, wherein the rendering the sub-region includes rendering the sub-region of the anatomical map responsively to the user input.

Further in accordance with an embodiment of the present disclosure, the method includes receiving user input of a speed of the rendering of the time-bounded portion of the propagation of the cardiac activation wave, wherein the rendering the sub-region includes rendering the time-bound portion of the propagation of the cardiac activation wave on the sub-region of the anatomical map responsively to the user input of the speed.

Still further in accordance with an embodiment of the present disclosure, the method includes a user interface to receive user input of a width of the cardiac activation wave, wherein the processing circuitry is configured to render to the display the time-bound portion of the propagation of the cardiac activation wave on the sub-region of the anatomical map responsively to the user input of the width of the cardiac activation wave.

There is also provided in accordance with still another embodiment of the present disclosure, a software product, including a non-transient computer-readable medium in which program instructions are stored, which instructions, when read by a central processing unit (CPU), cause the CPU to: identify local activation times (LATs) from electrical activity of tissue of a chamber of a heart captured by electrodes of a catheter inserted into the chamber and associate the LATs with respective positions on the surface of an anatomical map of the chamber of the heart; compute a propagation of a cardiac activation wave over the anatomical map of the chamber of the heart from a start time in a cardiac cycle to an end time in the cardiac cycle responsively to the captured electrical activity, wherein the propagation of the cardiac activation wave is calculated using a sliding window of the local activation times; render a sub-region of the anatomical map to a display; select a time-bounded portion of the propagation of the cardiac activation wave commencing at a time after the start time, wherein the time after the start time is the earliest time the wave would arrive in the sub-region of the anatomical map; and render the time-bounded portion of the propagation of the cardiac activation wave on the sub-region of the anatomical map to the display.

### BRIEF DESCRIPTION OF THE DRAWINGS

The present invention will be understood from the following detailed description, taken in conjunction with the drawings in which:
Fig. 1 is a schematic view of a medical procedure system constructed and operative in accordance with an exemplary embodiment of the present invention;
Fig. 2 is a schematic view of a catheter for use in the system of Fig. 1;
Fig. 3 is a schematic view illustrating propagation of a cardiac activation wave over an anatomical map generated by the system of Fig. 1;
Fig. 4 is a schematic view illustrating propagation of the cardiac activation wave of Fig. 3 on a sub-region of the anatomical map;
Fig. 5 is a schematic view illustrating rendering of a time-bound portion of the propagation of a cardiac activation wave on a sub-region on the inside of the anatomical map of Fig. 3; and
Fig. 6 is a flowchart including steps in a method of operation of the system of Fig. 1.

### DESCRIPTION OF EXAMPLE EMBODIMENTS

### OVERVIEW

As mentioned previously, in a two-step procedure, mapping followed by ablation, electrical activity at points within the heart is typically sensed and measured by advancing a catheter containing one or more electrodes into the heart, and acquiring data at a multiplicity of points. These data are then utilized to select the target areas at which the ablation is to be performed.

The mapping may be used to compute a propagation of a cardiac activation wave over a chamber of the heart from a start time in a cardiac cycle to an end time in the cardiac cycle responsively to captured electrical activity, such as local activation times (LATs). The propagation of the cardiac activation wave may be rendered to a display, typically in slow motion (or at any suitable selected speed), over an anatomical map of the chamber using colors and/or symbols. The propagation is then analyzed via a physician to determine if and where to ablate the tissue of the chamber of the heart.

The physician may be viewing a sub-region of the anatomical map, for example, the physician may want to zoom into a sub-region of the anatomical map or view a sub-region of the anatomical map from inside the anatomical map (e.g., from the viewpoint of a virtual camera in which only the sub-region of the map can be viewed at any one time). Assuming that the selected sub-region of the anatomical map is not at the start of the wave propagation, and the physician then runs the propagation of the cardiac activation wave over the anatomical map of the chamber of the heart, the physician will not see the propagation on the selected rendered sub-region of the anatomical map until after some delay, i.e., until the wave finally reaches the selected rendered sub-region of the anatomical map after "running" over the non-rendered portions of the map. In general, the above problem may be observed whenever the desired wave propagation range is smaller than the whole wave propagation range. If the wave propagation is run in a loop mode whereby the wave propagation runs from beginning to end and then repeats from the beginning etc., the delay may be even greater as the physician needs to wait while the wave propagation is running over the non-rendered portions of the anatomical map prior to, and after, the selected rendered sub-regions of the map currently being viewed. Once the wave propagation enters the selected rendered sub-region, the wave propagation disappears quickly until the next repeat of the wave propagation after another delay. The delay in the wave being visible in the selected sub-region of the anatomical map may be confusing to the physician and wastes valuable time during a cardiac procedure especially when the physician needs to repeatedly run the wave propagation in order to determine if and where to ablate the tissue of the chamber of the heart based on the wave propagation.

Embodiments of the present invention solve the above problems by automatically computing the earliest time (according to a cardiac cycle time of the wave propagation) that the wave would arrive in the selected sub-region of the anatomical map currently in view on a display. Rendering of the wave propagation to the display then starts from the computed earliest time in the cardiac cycle of the wave propagation. Therefore, upon the physician running the wave propagation, the wave propagation is automatically rendered without any substantial delay over the selected sub-region currently in view.

Similarly, the latest time (according to the cardiac cycle time of the wave propagation) when the wave would leave the sub-region currently in view may be computed and rendering of the wave propagation may be stopped at around that point. Therefore, when the wave propagation is run by the physician, the wave propagation may immediately start to render in the sub-region currently in view until the wave propagation leaves the sub-region, and be repeatedly rendered in the sub-region without any significant delay.

Embodiments of the present invention are useful whether the sub-region of the anatomical map is viewed from a viewpoint (e.g., a virtual camera) within the anatomical map or from a viewpoint outside the anatomic map.

In some embodiments, the physician may select a sub-region from the outside of the anatomical map by marking, or pointing to, a portion of the anatomical map. The selected sub-region is then optionally enlarged, or otherwise highlighted or marked, providing the physician with better visibility of the sub-region and the rendering of the wave propagation on the sub-region.

In some embodiments, the virtual camera may be manipulated by the physician using a suitable user interface (e.g., mouse, joystick or other pointing device) thereby selecting the sub-region of the anatomical map to be viewed on the display.

In some embodiments, parameters of the wave propagation rendering, such as rendering speed and/or wave width, may be selected by the physician to allow the physician to better understand the wave propagation in a given region. For example, if the physician selects a sub-region associated with fast wave propagation, the physician may slow down the wave propagation to carefully view the wave propagation in the sub-region. In some embodiments, the parameters may be automatically set according to any suitable parameters, for example, the size of the selected region, and/or the fraction of the size of the selected region compared to size of the region over which the whole wave propagation is originally computed.

### SYSTEM DESCRIPTION

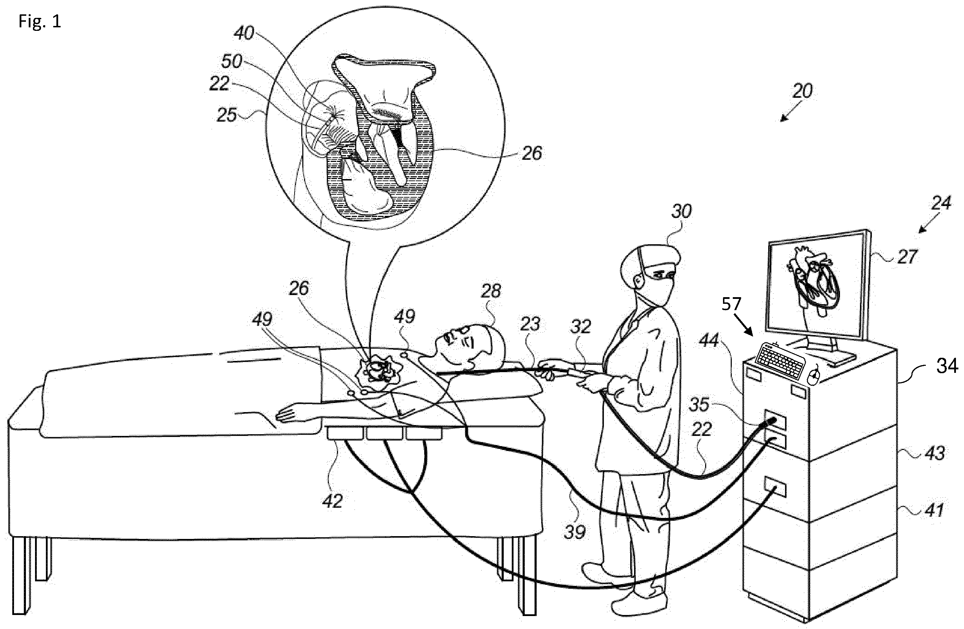

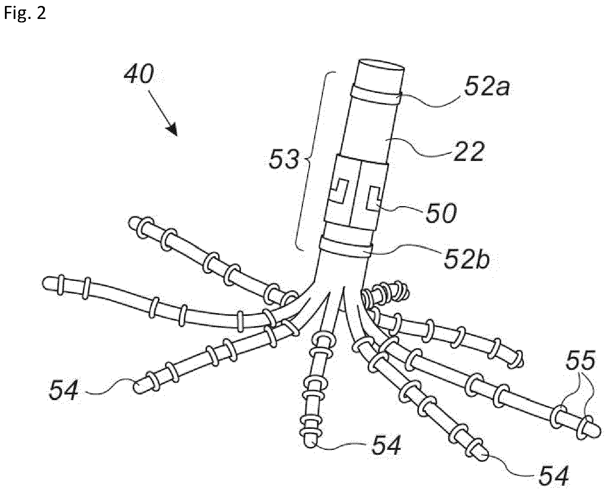

Reference is now made to Fig. 1, which is a schematic view of a medical procedure system 20 constructed and operative in accordance with an exemplary embodiment of the present invention. Reference is also made to Fig. 2, which is a schematic view of a catheter 40 for use in the system 20 of Fig. 1.

The medical procedure system 20 is used to determine the position of the catheter 40, seen in an inset 25 of Fig. 1 and in more detail in Fig. 2. The catheter 40 includes a shaft 22 and a plurality of flexible arms 54 (only some labeled for the sake of simplicity) having respective proximal ends connected to a distal end of the shaft 22. The catheter 40 is configured to be inserted into a body-part (e.g., a chamber of a heart 26) of a living subject.

The catheter 40 includes a position sensor 53 disposed on the shaft 22 in a predefined spatial relation to the proximal ends of the flexible arms 54. The position sensor 53 may include a magnetic sensor 50 and/or at least one shaft electrode 52. The magnetic sensor 50 may include at least one coil, for example, but not limited to, a dual-axis or a triple axis coil arrangement to provide position data for location and orientation including roll. The catheter 40 includes multiple electrodes 55 (only some are labeled in Fig. 2 for the sake of simplicity) disposed at respective locations along each of the flexible arms 54 and configured to capture electrical activity of tissue of a chamber of the heart 26 at respective positions in the heart 26 over time. Typically, the catheter 40 may be used for mapping electrical activity in the heart 26 of the living subject using the electrodes 55, or for performing any other suitable function in a body-part of a living subject.

The medical procedure system 20 may determine a position and orientation of the shaft 22 of the catheter 40 based on signals provided by the magnetic sensor 50 and/or the shaft electrodes 52 (proximal-electrode 52a and distal-electrode 52b) fitted on the shaft 22, on either side of the magnetic sensor 50. The proximal-electrode 52a, the distal-electrode 52b, the magnetic sensor 50 and at least some of the electrodes 55 are connected by wires running through the shaft 22 via a catheter connector 35 to various driver circuitries in a console 24. In some embodiments, at least two of the electrodes 55 of each of the flexible arms 54, the shaft electrodes 52, and the magnetic sensor 50 are connected to the driver circuitries in the console 24 via the catheter connector 35. In some embodiments, the distal electrode 52b and/or the proximal electrode 52a may be omitted.

The illustration shown in Fig. 2 is chosen purely for the sake of conceptual clarity. Other configurations of shaft electrodes 52 and electrodes 55 are possible. Additional functionalities may be included in the position sensor 53. Elements which are not relevant to the disclosed embodiments of the invention, such as irrigation ports, are omitted for the sake of clarity.

A physician 30 navigates the catheter 40 to a target location in a body part (e.g., heart 26) of a patient 28 by manipulating the shaft 22 using a manipulator 32 near the proximal end of the catheter 40 and/or deflection from a sheath 23. The catheter 40 is inserted through the sheath 23, with the flexible arms 54 gathered together, and only after the catheter 40 is retracted from the sheath 23, the flexible arms 54 are able to spread and regain their intended functional shape. By containing flexible arms 54 together, the sheath 23 also serves to minimize vascular trauma on its way to the target location.

Console 24 comprises processing circuitry 41, typically a general-purpose computer and a suitable front end and interface circuits 44 for generating signals in, and/or receiving signals from, body surface electrodes 49 which are attached by wires running through a cable 39 to the chest and to the back, or any other suitable skin surface, of the patient 28.

Console 24 further comprises a magnetic-sensing sub-system. The patient 28 is placed in a magnetic field generated by a pad containing at least one magnetic field radiator 42, which is driven by a unit 43 disposed in the console 24. The magnetic field radiator(s) 42 is/are configured to transmit alternating magnetic fields into a region where the body-part (e.g., the heart 26) is located. The magnetic fields generated by the magnetic field radiator(s) 42 generate direction signals in the magnetic sensor 50. The magnetic sensor 50 is configured to detect at least part of the transmitted alternating magnetic fields and provide the direction signals as corresponding electrical inputs to the processing circuitry 41.

In some embodiments, the processing circuitry 41 uses the position-signals received from the shaft electrodes 52, the magnetic sensor 50 and the electrodes 55 to estimate a position of the catheter 40 inside an organ, such as inside a cardiac chamber. In some embodiments, the processing circuitry 41 correlates the position signals received from the electrodes 52, 55 with previously acquired magnetic location-calibrated position signals, to estimate the position of the catheter 40 inside a cardiac chamber. The position coordinates of the shaft electrodes 52 and the electrodes 55 may be determined by the processing circuitry 41 based on, among other inputs, measured impedances, or on proportions of currents distribution, between the electrodes 52, 55 and the body surface electrodes 49. The console 24 drives a display 27, which shows the distal end of the catheter 40 inside an anatomical map of the heart 26.

The method of position sensing using current distribution measurements and/or external magnetic fields is implemented in various medical applications, for example, in the Carto^{®} system, produced by Biosense Webster Inc. (Irvine, California), and is described in detail in U.S. Patent Nos. 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publication Nos. 2002/0065455 A1, 2003/0120150 A1 and 2004/0068178 A1.

The Carto^{®}3 system applies an Active Current Location (ACL) impedance-based position-tracking method. In some embodiments, using the ACL method, the processing circuitry 41 is configured to create a mapping (e.g., current-position matrix (CPM)) between indications of electrical impedance and positions in a magnetic coordinate frame of the magnetic field radiator(s) 42. The processing circuitry 41 estimates the positions of the shaft electrodes 52 and the electrodes 55 by performing a lookup in the CPM.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The system 20 also includes a user interface 57 to receive user input such as a selection of a sub-region of the anatomical map for display, manipulation of a virtual camera used in viewing the sub-region of the anatomical map, a rendering speed of a wave propagation of a cardiac activation wave, and/or a width of a cardiac activation wave being rendered. The user interface 57 may include a keyboard and/or touch screen, a foot pedal, and optionally a pointing device such as a mouse, stylus and/or joystick.

Fig. 1 shows only elements related to the disclosed techniques, for the sake of simplicity and clarity. The system 20 typically comprises additional modules and elements that are not directly related to the disclosed techniques, and thus are intentionally omitted from Fig. 1 and from the corresponding description.

The catheter 40 described above includes eight flexible arms 54 with six electrodes 55 per arm 54. Any suitable catheter may be used instead of the catheter 40, for example, a catheter with a different number of flexible arms and/or electrodes per arm, or a different probe shape such as a balloon catheter or a lasso catheter, by way of example only.

The medical procedure system 20 may also perform ablation of heart tissue using any suitable catheter, for example using the catheter 40 or a different catheter and any suitable ablation method. The console 24 may include an RF signal generator 34 configured to generate RF power to be applied by an electrode or electrodes of a catheter connected to the console 24, and one or more of the body surface electrodes 49, to ablate a myocardium of the heart 26. The console 24 may include a pump (not shown), which pumps irrigation fluid into an irrigation channel to a distal end of a catheter performing ablation. The catheter performing the ablation may also include temperature sensors (not shown) which are used to measure a temperature of the myocardium during ablation and regulate an ablation power and/or an irrigation rate of the pumping of the irrigation fluid according to the measured temperature.

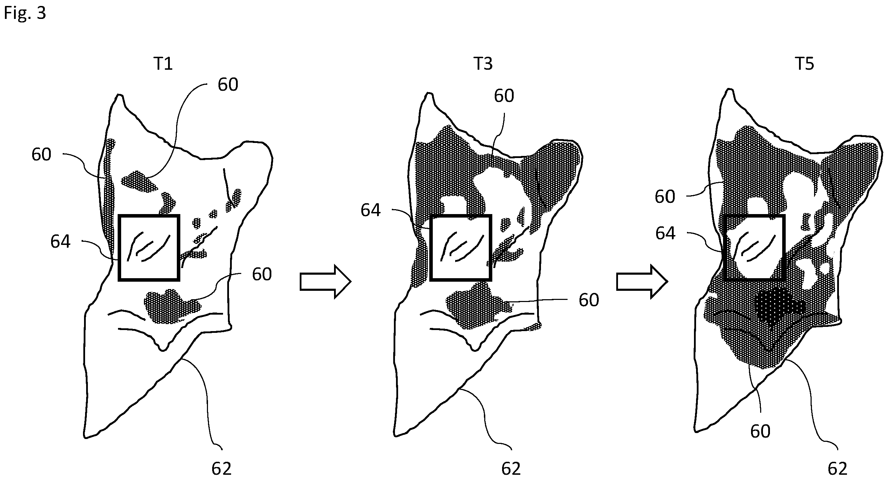

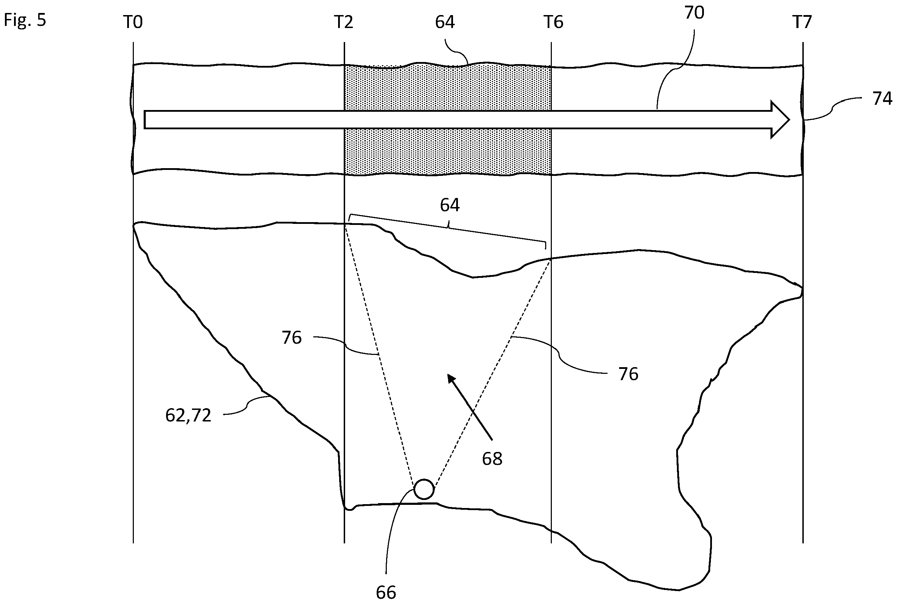

Reference is now made to Fig. 3, which is a schematic view illustrating propagation of a cardiac activation wave 60 over an anatomical map 62 generated by the system 20 of Fig. 1. The processing circuitry 41 is configured to compute a propagation of the cardiac activation wave 60 over the anatomical map 62 of a chamber of the heart 26 from a start time in a cardiac cycle to an end time in the cardiac cycle responsively to electrical activity captured by the electrodes 55 (Fig. 2). The propagation starts at time T0 and continues until an end time T7 as described in more detail with reference to Fig. 5. Fig. 3 shows the progression of the cardiac activation wave 60 over the anatomical map 62 at times T1, T3 and T5. The cardiac activation wave 60 is represented by shaded portions moving over the anatomical map 62.

The anatomical map 62 may be generated using any suitable anatomical map generation method, for example, but not limited to, Fast Anatomical Mapping (FAM). FAM is described in US Patent No. 10,918,310 to Cohen, et al. In FAM, a smooth shell is generated around a three-dimensional (3D) cloud of data points, such as a cloud of computed electrode positions of the electrodes 55. The propagation of the cardiac activation wave 60 may be computed using any suitable method, for example, but not limited to, one or more of the methods disclosed in U.S. Patent Nos. 10,136,828, 6,226,542, 6,301,496, and in 6,892,091.

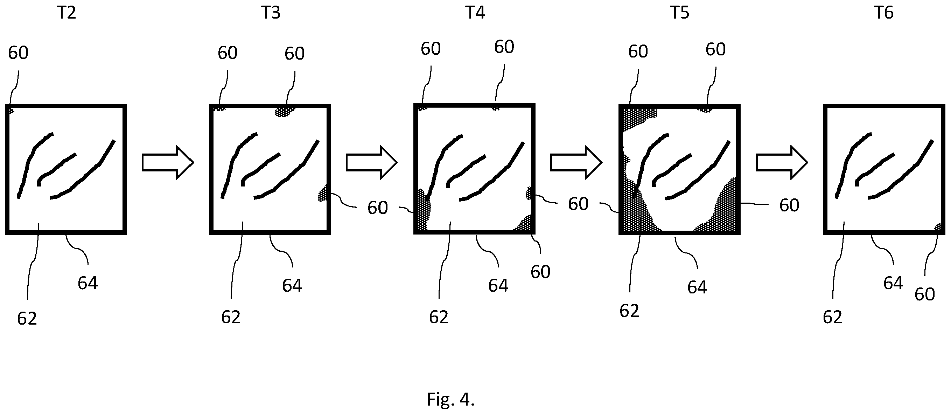

Fig. 3 shows a square sub-region 64 on the anatomical map 62. The sub-region 64 is selected by the physician 30 and enlarged as shown in Fig. 4. Fig. 3 shows that at time T1, the cardiac activation wave 60 has not yet appeared in the sub-region 64. Fig. 3 shows that at time T3, the cardiac activation wave 60 has already appeared in the sub-region 64 and that presence continues to grow through to time T5.

Reference is now made to Fig. 4, which is a schematic view illustrating propagation of the cardiac activation wave 60 of Fig. 3 on the sub-region 64 of the anatomical map 62 at times T2 to T6.

The processing circuitry 41 is configured to render to the display 27 the sub-region 64 of the anatomical map 62 of a chamber of the heart 26. The processing circuitry 41 is configured to select a time-bounded portion of the propagation of the cardiac activation wave 60 commencing at a time (referred herein as "adjusted start time") after the cycle start time T0 (and optionally ending at a time (referred herein as "adjusted end time") before the cycle end time T7) responsively to when the propagation would commence to be rendered in the sub-region 64 of the anatomical map 62 (and optionally when the propagation of the cardiac activation wave 60 would complete to be rendered in the sub-region 64 of the anatomical map 62). Therefore, the processing circuitry 41 is configured to compute the adjusted start time and optionally the adjusted end time for rendering the propagation of the cardiac activation wave 60 according to when the propagation would be rendered in the sub-region 64. Therefore, the time-bounded portion is defined by the adjusted start time and optionally the adjusted end time. In the example of Fig. 4, the time bound portion has an adjusted start time of T2 (compared to the original cycle start time of T0) and an adjusted end time of T6 (compared to the original cycle end time of T7).

The processing circuitry 41 is configured to render to the display 27 the time-bound portion of the propagation of the cardiac activation wave 60 on the sub-region 64 of the anatomical map 62 by rendering the generated propagation of the cardiac activation wave 60 from the adjusted start time (e.g., T2) to the adjusted end time (e.g., T6). The time-bound portion of the propagation of the cardiac activation wave 60 may be repeatedly rendered from the adjusted start time (e.g., T2) to the adjusted end time (e.g., T6) in loop-mode thereby enabling the physician 30 to carefully inspect the propagation of the wave over the sub-region 64.

The anatomical map 62 and the sub-region 64 shown in Figs. 3 and 4 are typically viewed from a viewpoint outside of the anatomical map 62. However, the sub-region 64 may also be viewed from a viewpoint (e.g., virtual camera) within the anatomical map 62, as described now is more detail with reference to Fig. 5.

Reference is now made to Fig. 5, which is a schematic view illustrating rendering of the time-bound portion of the propagation of the cardiac activation wave 60 on the sub-region 64 within the anatomical map 62 of Fig. 3.

The anatomical map 62 may be viewed from the viewpoint of a virtual camera 66 within the anatomical map 62 so that a field of view 68 of the virtual camera 66 is rendered to the display 27. The field of view 68 is delineated by two dotted lines 76. Fig. 5 shows a cross-section 72 of the anatomical map 62, with the virtual camera 66 positioned therein, and a view of an internal wall 74 of the interior of the anatomical map 62 as seen from the virtual camera 66. A portion of the internal wall 74 has been shaded to indicate the portion of the internal wall 74 being viewed by the virtual camera 66 and corresponds to the sub-region 64 of the anatomical map 62. The regions to the left and right of the sub-region 64 could be viewed by moving the virtual camera 66 left and right, respectively.

An arrow 70 shows how the cardiac activation wave 60 propagates over the internal wall 74 of the anatomical map 62 from time T0 to time T7. It can be seen that the cardiac activation wave 60 enters the sub-region 64 at time T2 and leaves the sub-region 64 at time T6. Therefore, when the physician 30 selects running the propagation of the cardiac activation wave 60 while the sub-region 64 is being viewed from the virtual camera 66, the propagation is rendered from time T2 until time T6 and not during the time periods T0 to T2 and T6 to T7.

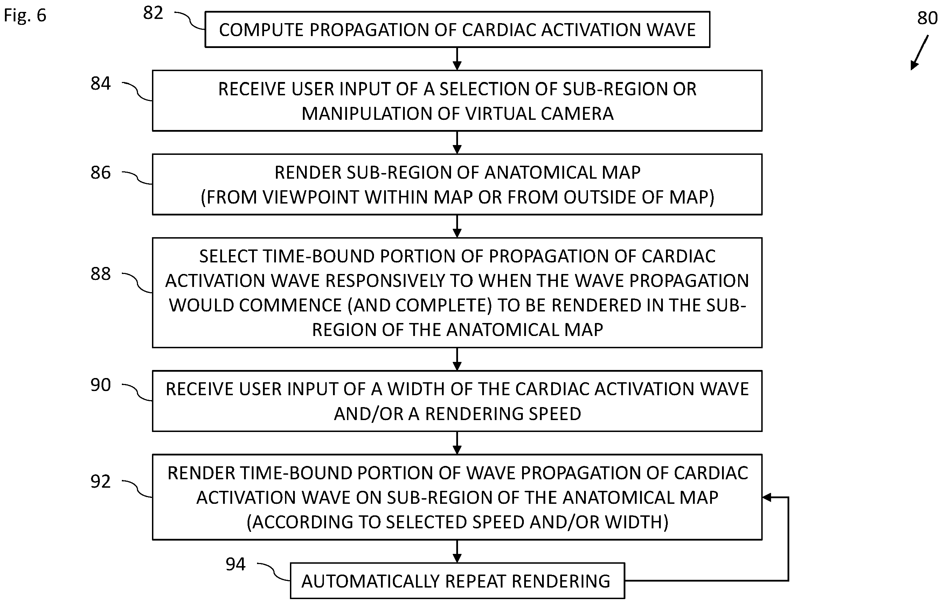

Reference is now made to Fig. 6, which is a flowchart 80 including steps in a method of operation of the system 20 of Fig. 1. Reference is also made to Fig. 5.

The processing circuitry 41 is configured to generate the anatomical map 62 of the chamber of the heart 26 and render the anatomical map 62 or a part thereof to the display 27. The processing circuitry 41 is configured to compute (block 82) a propagation of the cardiac activation wave 60 over the anatomical map 62 of a chamber of the heart 26 (Fig. 1) from a start time in a cardiac cycle to an end time in the cardiac cycle of the cardiac activation wave 60 responsively to electrical activity captured by the electrodes 55 (Fig. 2). The propagation of the cardiac activation wave 60 may be computed using any suitable method. In some embodiments, local activations times (LATs) are identified from the cardiac electrical activity signals (e.g., electrocardiograms (ECGs) or intracardiac electrograms (IEGMs)) and associated with respective positions on the surface of the anatomical map 62. The propagation of the cardiac activation wave 60 may then computed based on the LATs using a sliding window of LATs. For example, at time T0 LATs in a range of -200ms to -160ms are rendered on the anatomical map 62, at time T1 LATs in a range -180ms to -140ms are rendered on the anatomical map 62, and at a time T2 LATs in a range -160ms to -120ms are rendered on the anatomical map 62, and so on. The LAT window continues to move until a complete cycle of the cardiac activation wave cardiac activation wave 60 has been rendered over the anatomical map 62. In this way, the cardiac activation wave 60 is seen to move over the surface of the anatomical map 62. The width of the sliding window, i.e., the range of LATs in the sliding window may be configurable. Color or shading may be used to indicate the LATs being rendered at any one time in the propagation. Different colors or shading may be used to indicate different LAT values. For example, LATs in the range of -200ms to -160ms may be rendered on the anatomical map 62 in red, LATs in the range -180ms to -140ms may be rendered on the anatomical map 62 in orange, LATs in a range -160ms to -120ms may be rendered on the anatomical map 62 in yellow, and so on.

In some embodiments, the user interface 57 is configured to receive user input (block 84) of a selection of the sub-region 64 of the anatomical map 62 of the chamber of the heart 26. The sub-region 64 may be selected by the physician 30 from the surface of the anatomical map 62, for example, by selecting a region or point on the map 62, e.g., using a pointing device or a touch sensitive screen. The processing circuitry 41 is configured to render (block 86) the sub-region 64 of the anatomical map 62 responsively to the user input. In some embodiments, the processing circuitry 41 is configured to render the sub-region 64 of the anatomical map 62 from a viewpoint outside of the anatomical map 62.

In some embodiments, the user interface 57 is configured to receive (block 84) user input of a manipulation of the virtual camera 66 to change a rendered view of the anatomical map 62 from within the anatomical map 62 to a view of the sub-region 64 from within the anatomical map 62. The processing circuitry 41 is configured to render (block 86) to the display 27 the sub-region 64 of the anatomical map 62 from a viewpoint (e.g., the virtual camera 66) within the anatomical map 62 responsively to the user input of the manipulation of the virtual camera 66.

The processing circuitry 41 is configured to select (block 88) a time-bounded portion of the propagation of the cardiac activation wave 60 commencing at a time (e.g., an adjusted start time T2) after the cycle start time (e.g., T0) responsively to when the propagation would commence to be rendered in the sub-region 64 of the anatomical map 62. In some embodiments, the processing circuitry 41 is configured to select the time-bounded portion of the propagation of the cardiac activation wave 60 ending at a time (e.g., adjusted end time T6) before the cycle end time (e.g., T7) responsively to when the propagation of the cardiac activation wave 60 would complete to be rendered in the sub-region 64 of the anatomical map 62.

The user interface 57 may be configured to receive (block 90) user input of a rendering speed of the time-bounded portion of the propagation of the cardiac activation wave 60 and/or a width of the cardiac activation wave 60 over the sub-region 64 of the anatomical map 62. The time-bound portion may be rendered at real-time speed (i.e., at the speed the cardiac activation wave 60 propagates over the chamber of the heart 26) or slow or faster than real-time speed. The width of the cardiac activation wave 60 provides a measure of the LAT values included in the sliding window of the propagation of the cardiac activation wave 60 over the sub-region 64 of the anatomical map 62 at any one time. For example, if the selected width is 40 milliseconds, the range of LAT values shown on the anatomical map 62 at any one time is within a range width of 40 milliseconds (ms) (e.g., from -100 ms to -60 ms at time T2, or from 10 ms to 50 ms at time T6, etc.) and as the cardiac activation wave 60 propagates over the anatomical map 62, the sliding window of LAT values is constantly shifted but has a static width of 40 ms.

The processing circuitry 41 is configured to render (block 92) to the display 27 the time-bound portion of the propagation of the cardiac activation wave 60 on the sub-region 64 of the anatomical map 62. In other words, the processing circuitry 41 is configured to render to the display 27 the propagation of the cardiac activation wave 60 from the adjusted start time (until the adjusted end time). In some embodiments, the processing circuitry 41 is configured to render the sub-region 64 of the anatomical map 62 from a viewpoint (e.g., from the virtual camera 66) within the anatomical map 62. In some embodiments, the processing circuitry 41 is configured to render the sub-region 64 of the anatomical map 62 from a viewpoint (e.g., from the virtual camera 66) within the anatomical map 62, while the viewpoint (e.g., the virtual camera 66) is static during rendering of the time-bound portion of the propagation of the cardiac activation wave 60 on the sub-region 64 of the anatomical map 62. In some embodiments, the processing circuitry 41 is configured to render the sub-region 64 of the anatomical map 62 from a viewpoint outside of the anatomical map 62.

In some embodiments, the processing circuitry 41 is configured to render to the display 27 the time-bound portion of the propagation of the cardiac activation wave 60 on the sub-region 64 of the anatomical map 62 responsively to the user input of the rendering speed at the step of block 90. In some embodiments, the processing circuitry 41 is configured to render to the display 27 the time-bound portion of the propagation of the cardiac activation wave 60 on the sub-region 64 of the anatomical map 62 responsively to the user input of the width of the cardiac activation wave 60 at the step of block 90.

The processing circuitry 41 may be configured to automatically repeat (block 94) rendering of the time-bound portion of the propagation of the cardiac activation wave 60 on the sub-region 64 of the anatomical map 62.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and sub-combinations of the various features described hereinabove, as well as variations and modifications thereof which would occur to persons skilled in the art upon reading the foregoing description and which are not disclosed in the prior art.

## Claims

1. A medical system (20) for wave propagation enhancement, the medical system comprising:
a catheter (40) configured to be inserted into a chamber of a heart (26), and including electrodes (52, 55) configured to capture electrical activity of tissue of the chamber over time;
a display (27); and
processing circuitry (41) configured to:
identify local activation times (LATs) from the captured electrical activity and associate the local activation times with respective positions on the surface of an anatomical map (62) of the chamber;
compute a propagation of a cardiac activation wave (60) over the anatomical map of the chamber from a start time in a cardiac cycle to an end time in the cardiac cycle responsively to the captured electrical activity, wherein the propagation of the cardiac activation wave is calculated using a sliding window of the local activation times;
render to the display a sub-region (64) of the anatomical map;
select a time-bounded portion of the propagation of the cardiac activation wave commencing at a time after the start time, wherein the time after the start time is the earliest time the wave would arrive in the sub-region of the anatomical map; and
render to the display the time-bounded portion of the propagation of the cardiac activation wave on the sub-region of the anatomical map.

2. The system according to claim 1, wherein the processing circuitry is configured to select the time-bounded portion of the propagation of the cardiac activation wave ending at a time before the end time responsively to when the propagation of the cardiac activation wave would complete to be rendered in the sub-region of the anatomical map.

3. The system according to claim 2, wherein the processing circuitry is configured to automatically repeat rendering of the time-bounded portion of the propagation of the cardiac activation wave on the sub-region of the anatomical map.

4. The system according to claim 1, wherein the processing circuitry is configured to render the sub-region of the anatomical map from a viewpoint within the anatomical map.

5. The system according to claim 4, wherein the processing circuitry is configured to render the sub-region of the anatomical map from a viewpoint within the anatomical map, while the viewpoint is static during rendering of the time-bounded portion of the propagation of the cardiac activation wave on the sub-region of the anatomical map.

6. The system according to claim 4, further comprising a user interface to receive user input of a manipulation of a virtual camera to change a rendered view of the anatomical map from within the anatomical map, wherein the processing circuitry is configured to render the sub-region of the anatomical map responsively to the user input.

7. The system according to claim 1, wherein the processing circuitry is configured to render the sub-region of the anatomical map from a viewpoint outside of the anatomical map.

8. The system according to claim 7, further comprising a user interface to receive user input of a selection of the sub-region of the anatomical map, wherein the processing circuitry is configured to render the sub-region of the anatomical map responsively to the user input.

9. The system according to claim 1, further comprising a user interface to receive user input of a speed of the rendering of the time-bounded portion of the propagation of the cardiac activation wave, wherein the processing circuitry is configured to render to the display the time-bounded portion of the propagation of the cardiac activation wave on the sub-region of the anatomical map responsively to the user input of the speed.

10. The system according to claim 1, further comprising a user interface to receive user input of a width of the cardiac activation wave, wherein the processing circuitry is configured to render to the display the time-bounded portion of the propagation of the cardiac activation wave on the sub-region of the anatomical map responsively to the user input of the width of the cardiac activation wave.

11. A medical method for wave propagation control enhancement, the method comprising:
identifying local activation times (LATs) from electrical activity of tissue of a chamber of a heart (26) captured by electrodes (52, 55) of a catheter (40) inserted into the chamber and associating the local activation times with respective positions on the surface of an anatomical map (62) of the chamber of the heart;
computing a propagation of a cardiac activation wave (60) over the anatomical map of the chamber of the heart from a start time in a cardiac cycle to an end time in the cardiac cycle responsively to the captured electrical activity, wherein the propagation of the cardiac activation wave is calculated using a sliding window of the local activation times;
rendering a sub-region (64) of the anatomical map to a display (27);
selecting a time-bounded portion of the propagation of the cardiac activation wave commencing at a time after the start time, wherein the time after the start time is the earliest time the wave would arrive in the sub-region of the anatomical map; and
rendering the time-bounded portion of the propagation of the cardiac activation wave on the sub-region of the anatomical map to the display.

12. The method according to claim 11, wherein the selecting includes selecting the time-bounded portion of the propagation of the cardiac activation wave ending at a time before the end time responsively to when the propagation of the cardiac activation wave would complete to be rendered in the sub-region of the anatomical map.

13. The method according to claim 12, further comprising automatically repeating rendering of the time-bounded portion of the propagation of the cardiac activation wave on the sub-region of the anatomical map.

14. The method according to claim 11, wherein the rendering the sub-region includes rendering the sub-region of the anatomical map from a viewpoint within the anatomical map.

15. The method according to claim 14, wherein the rendering the sub-region includes rendering the sub-region of the anatomical map from a viewpoint within the anatomical map, while the viewpoint is static during rendering of the time-bounded portion of the propagation of the cardiac activation wave on the sub-region of the anatomical map.

16. The method according to claim 14, further comprising receiving user input of a manipulation of a virtual camera to change a rendered view of the anatomical map from within the anatomical map, wherein the rendering the sub-region includes rendering the sub-region of the anatomical map responsively to the user input.

17. The method according to claim 11, wherein the rendering the sub-region includes rendering the sub-region of the anatomical map from a viewpoint outside of the anatomical map.

18. The method according to claim 17, further comprising receiving user input of a selection of the sub-region of the anatomical map, wherein the rendering the sub-region includes rendering the sub-region of the anatomical map responsively to the user input.

19. The method according to claim 11, further comprising receiving user input of a speed of the rendering of the time-bounded portion of the propagation of the cardiac activation wave, wherein the rendering the sub-region includes rendering the time-bounded portion of the propagation of the cardiac activation wave on the sub-region of the anatomical map responsively to the user input of the speed.

20. The method according to claim 11, further comprising a user interface to receive user input of a width of the cardiac activation wave, wherein the processing circuitry is configured to render to the display the time-bounded portion of the propagation of the cardiac activation wave on the sub-region of the anatomical map responsively to the user input of the width of the cardiac activation wave.

21. A software product, comprising a non-transient computer-readable medium in which program instructions are stored, which instructions, when read by a central processing unit (CPU) (41), cause the CPU to:
identify local activation times (LATs) from electrical activity of tissue of a chamber of a heart (26) captured by electrodes (52, 55) of a catheter (40) inserted into the chamber and associate the LATs with respective positions on the surface of an anatomical map (62) of the chamber of the heart;
compute a propagation of a cardiac activation wave (60) over the anatomical map of the chamber of the heart from a start time in a cardiac cycle to an end time in the cardiac cycle responsively to the captured electrical activity, wherein the propagation of the cardiac activation wave is calculated using a sliding window of the local activation times;
render a sub-region (64) of the anatomical map to a display (27);
select a time-bounded portion of the propagation of the cardiac activation wave commencing at a time after the start time, wherein the time after the start time is the earliest time the wave would arrive in the sub-region of the anatomical map; and
render the time-bounded portion of the propagation of the cardiac activation wave on the sub-region of the anatomical map to the display.

## Patentansprüche

1. Medizinisches System (20) zur Verbesserung der Wellenausbreitung, das medizinische System umfassend:
einen Katheter (40), der so konfiguriert ist, dass er in eine Herzkammer (26) eingeführt werden kann, und der Elektroden (52, 55) einschließt, die so konfiguriert sind, dass sie die elektrische Aktivität von Gewebe der Kammer über die Zeit erfassen;
eine Anzeige (27); und
Verarbeitungsschaltlogik (41) konfiguriert zum:
Identifizieren lokaler Aktivierungszeiten (LATs) aus der erfassten elektrischen Aktivität und Zuordnen der lokalen Aktivierungszeiten zu den jeweiligen Positionen auf der Oberfläche einer anatomischen Karte (62) der Kammer;
Berechnen einer Ausbreitung einer kardialen Aktivierungswelle (60) über die anatomische Karte der Kammer von einer Startzeit in einem Herzzyklus zu einer Endzeit in dem Herzzyklus in Reaktion auf die erfasste elektrische Aktivität, wobei die Ausbreitung der kardialen Aktivierungswelle unter Verwendung eines gleitenden Fensters der lokalen Aktivierungszeiten berechnet wird;
Darstellen einer Teilregion (64) der anatomischen Karte auf der Anzeige;
Auswählen eines zeitlich begrenzten Abschnitts der Ausbreitung der kardialen Aktivierungswelle, der zu einer Zeit nach der Startzeit beginnt, wobei die Zeit nach der Startzeit der früheste Zeitpunkt ist, zu dem die Welle in der Teilregion der anatomischen Karte ankommen würde; und
Darstellen des zeitlich begrenzten Abschnitts der Ausbreitung der kardialen Aktivierungswelle in der Teilregion der anatomischen Karte auf der Anzeige.

2. System nach Anspruch 1, wobei die Verarbeitungsschaltlogik konfiguriert ist, um den zeitlich begrenzten Abschnitt der Ausbreitung der Herzaktivierungswelle auszuwählen, der zu einer Zeit vor der Endzeit endet, die darauf reagiert, wann die Ausbreitung der Herzaktivierungswelle abgeschlossen wäre, um in der Teilregion der anatomischen Karte dargestellt zu werden.

3. System nach Anspruch 2, wobei die Verarbeitungsschaltlogik konfiguriert ist, um das Darstellen des zeitlich begrenzten Abschnitts der Ausbreitung der kardialen Aktivierungswelle auf der Teilregion der anatomischen Karte automatisch zu wiederholen.

4. System nach Anspruch 1, wobei die Verarbeitungsschaltlogik konfiguriert ist, um die Teilregion der anatomischen Karte von einem Blickpunkt innerhalb der anatomischen Karte darzustellen.

5. System nach Anspruch 4, wobei die Verarbeitungsschaltlogik konfiguriert ist, um die Teilregion der anatomischen Karte von einem Blickpunkt innerhalb der anatomischen Karte darzustellen, während der Blickpunkt während der Darstellung des zeitlich begrenzten Abschnitts der Ausbreitung der Herzaktivierungswelle auf der Teilregion der anatomischen Karte statisch ist.

6. System nach Anspruch 4, ferner umfassend eine Benutzerschnittstelle, um eine Benutzereingabe einer Manipulation einer virtuellen Kamera zu empfangen, um eine dargestellte Ansicht der anatomischen Karte von innerhalb der anatomischen Karte zu ändern, wobei die Verarbeitungsschaltlogik so konfiguriert ist, dass sie die Teilregion der anatomischen Karte in Reaktion auf die Benutzereingabe darstellt.

7. System nach Anspruch 1, wobei die Verarbeitungsschaltlogik konfiguriert ist, um die Teilregion der anatomischen Karte von einem Blickpunkt außerhalb der anatomischen Karte darzustellen.

8. System nach Anspruch 7, ferner umfassend eine Benutzerschnittstelle, um eine Benutzereingabe einer Auswahl der Teilregion der anatomischen Karte zu empfangen, wobei die Verarbeitungsschaltlogik so konfiguriert ist, dass sie die Teilregion der anatomischen Karte in Reaktion auf die Benutzereingabe darstellt.

9. System nach Anspruch 1, ferner umfassend eine Benutzerschnittstelle, um eine Benutzereingabe einer Geschwindigkeit der Darstellung des zeitlich begrenzten Abschnitts der Ausbreitung der Herzaktivierungswelle zu empfangen, wobei die Verarbeitungsschaltlogik so konfiguriert ist, dass sie den zeitlich begrenzten Abschnitt der Ausbreitung der Herzaktivierungswelle auf der Teilregion der anatomischen Karte in Reaktion auf die Benutzereingabe der Geschwindigkeit auf der Anzeige darstellt.

10. System nach Anspruch 1, ferner umfassend eine Benutzerschnittstelle, um eine Benutzereingabe einer Breite der Herzaktivierungswelle zu empfangen, wobei die Verarbeitungsschaltlogik so konfiguriert ist, dass sie den zeitlich begrenzten Abschnitt der Ausbreitung der Herzaktivierungswelle auf der Teilregion der anatomischen Karte in Reaktion auf die Benutzereingabe der Breite der Herzaktivierungswelle auf der Anzeige darstellt.

11. Medizinisches Verfahren zur Verbesserung der Steuerung der Wellenausbreitung, das Verfahren umfassend:
Identifizieren von lokalen Aktivierungszeiten (LATs) aus der elektrischen Aktivität von Gewebe einer Herzkammer (26), die durch Elektroden (52, 55) eines in die Kammer eingeführten Katheters (40) erfasst wird, und Zuordnen der lokalen Aktivierungszeiten zu entsprechenden Positionen auf der Oberfläche einer anatomischen Karte (62) der Kammer des Herzens;
Berechnen einer Ausbreitung einer kardialen Aktivierungswelle (60) über die anatomische Karte der Herzkammer von einer Startzeit in einem Herzzyklus zu einer Endzeit in dem Herzzyklus in Reaktion auf die erfasste elektrische Aktivität, wobei die Ausbreitung der kardialen Aktivierungswelle unter Verwendung eines gleitenden Fensters der lokalen Aktivierungszeiten berechnet wird;
Darstellen einer Teilregion (64) der anatomischen Karte auf einer Anzeige (27);
Auswählen eines zeitlich begrenzten Abschnitts der Ausbreitung der kardialen Aktivierungswelle, der zu einer Zeit nach der Startzeit beginnt, wobei die Zeit nach der Startzeit der früheste Zeitpunkt ist, zu dem die Welle in der Teilregion der anatomischen Karte ankommen würde; und
Darstellen des zeitlich begrenzten Abschnitts der Ausbreitung der kardialen Aktivierungswelle in der Teilregion der anatomischen Karte auf der Anzeige.

12. Verfahren nach Anspruch 11, wobei das Auswählen das Auswählen des zeitlich begrenzten Abschnitts der Ausbreitung der Herzaktivierungswelle einschließt, der zu einer Zeit vor der Endzeit endet, die darauf reagiert, wann die Ausbreitung der Herzaktivierungswelle abgeschlossen wäre, um in der Teilregion der anatomischen Karte dargestellt zu werden.

13. Verfahren nach Anspruch 12, ferner umfassend das automatische Wiederholen des Darstellens des zeitlich begrenzten Abschnitts der Ausbreitung der Herzaktivierungswelle auf der Teilregion der anatomischen Karte.

14. Verfahren nach Anspruch 11, wobei das Darstellen der Teilregion das Darstellen der Teilregion der anatomischen Karte von einem Blickpunkt innerhalb der anatomischen Karte einschließt.

15. Verfahren nach Anspruch 14, wobei das Darstellen der Teilregion das Darstellen der Teilregion der anatomischen Karte von einem Blickpunkt innerhalb der anatomischen Karte einschließt, während der Blickpunkt während des Darstellens des zeitlich begrenzten Abschnitts der Ausbreitung der kardialen Aktivierungswelle auf der Teilregion der anatomischen Karte statisch ist.

16. Verfahren nach Anspruch 14, ferner umfassend das Empfangen der Benutzereingabe einer Manipulation einer virtuellen Kamera, um eine dargestellte Ansicht der anatomischen Karte von innerhalb der anatomischen Karte zu ändern, wobei das Darstellen der Teilregion das Darstellen der Teilregion der anatomischen Karte in Reaktion auf die Benutzereingabe einschließt.

17. Verfahren nach Anspruch 11, wobei das Darstellen der Teilregion das Darstellen der Teilregion der anatomischen Karte von einem Blickpunkt außerhalb der anatomischen Karte einschließt.

18. Verfahren nach Anspruch 17, ferner umfassend das Empfangen einer Benutzereingabe einer Auswahl der Teilregion der anatomischen Karte, wobei das Darstellen der Teilregion das Darstellen der Teilregion der anatomischen Karte in Reaktion auf die Benutzereingabe einschließt.

19. Verfahren nach Anspruch 11, ferner umfassend das Empfangen einer Benutzereingabe einer Geschwindigkeit des Darstellens des zeitlich begrenzten Abschnitts der Ausbreitung der Herzaktivierungswelle, wobei das Darstellen der Teilregion das Darstellen des zeitlich begrenzten Abschnitts der Ausbreitung der Herzaktivierungswelle auf der Teilregion der anatomischen Karte in Reaktion auf die Benutzereingabe der Geschwindigkeit umfasst.

20. Verfahren nach Anspruch 11, ferner umfassend eine Benutzerschnittstelle, um eine Benutzereingabe einer Breite der Herzaktivierungswelle zu empfangen, wobei die Verarbeitungsschaltlogik so konfiguriert ist, dass sie den zeitlich begrenzten Abschnitt der Ausbreitung der Herzaktivierungswelle auf der Teilregion der anatomischen Karte in Reaktion auf die Benutzereingabe der Breite der Herzaktivierungswelle auf der Anzeige darstellt.

21. Softwareprodukt, das ein nicht flüchtiges computerlesbares Medium umfasst, in dem Programmanweisungen gespeichert sind, wobei die Anweisungen, wenn sie von einer zentralen Verarbeitungseinheit (CPU) (41) gelesen werden, die CPU veranlassen zum:
Identifizieren von lokalen Aktivierungszeiten (LATs) aus der elektrischen Aktivität von Gewebe einer Herzkammer (26), die durch Elektroden (52, 55) eines in die Kammer eingeführten Katheters (40) erfasst wurde, und Zuordnen der LATs zu entsprechenden Positionen auf der Oberfläche einer anatomischen Karte (62) der Herzkammer;
Berechnen einer Ausbreitung einer kardialen Aktivierungswelle (60) über die anatomische Karte der Herzkammer von einer Startzeit in einem Herzzyklus zu einer Endzeit in dem Herzzyklus in Reaktion auf die erfasste elektrische Aktivität, wobei die Ausbreitung der kardialen Aktivierungswelle unter Verwendung eines gleitenden Fensters der lokalen Aktivierungszeiten berechnet wird;
Darstellen einer Teilregion (64) der anatomischen Karte auf einer Anzeige (27);
Auswählen eines zeitlich begrenzten Abschnitts der Ausbreitung der kardialen Aktivierungswelle, der zu einer Zeit nach der Startzeit beginnt, wobei die Zeit nach der Startzeit der früheste Zeitpunkt ist, zu dem die Welle in der Teilregion der anatomischen Karte ankommen würde; und
Darstellen des zeitlich begrenzten Abschnitts der Ausbreitung der kardialen Aktivierungswelle in der Teilregion der anatomischen Karte auf der Anzeige.

## Revendications

1. Système médical (20) d'amélioration de propagation d'onde, le système médical comprenant :
un cathéter (40) conçu pour être inséré dans une cavité d'un coeur (26) et comportant des électrodes (52, 55) configurées pour capter une activité électrique de tissu de la cavité au cours du temps ;
un dispositif d'affichage (27) ; et
un ensemble de circuits de traitement (41) configuré pour :
identifier des temps d'activation locale (LAT) à partir de l'activité électrique captée et associer les temps d'activation locale à des positions respectives sur la surface d'une carte anatomique (62) de la cavité ;
calculer une propagation d'une onde d'activation cardiaque (60) sur la carte anatomique de la cavité à partir d'un moment de début d'un cycle cardiaque jusqu'à un moment de fin du cycle cardiaque en réponse à l'activité électrique captée, dans lequel la propagation de l'onde d'activation cardiaque est calculée à l'aide d'une fenêtre glissante des temps d'activation locale ;
restituer sur le dispositif d'affichage une sous-région (64) de la carte anatomique ;
sélectionner une partie limitée dans le temps de la propagation de l'onde d'activation cardiaque commençant à un moment postérieur au moment de début, dans lequel le moment postérieur au moment de début est le moment le plus tôt où l'onde arriverait dans la sous-région de la carte anatomique ; et
restituer sur le dispositif d'affichage la partie limitée dans le temps de la propagation de l'onde d'activation cardiaque sur la sous-région de la carte anatomique.

2. Système selon la revendication 1, dans lequel l'ensemble de circuits de traitement est configuré pour sélectionner la partie limitée dans le temps de la propagation de l'onde d'activation cardiaque se terminant à un moment antérieur au moment de fin en réponse au moment où la propagation de l'onde d'activation cardiaque s'achèverait pour être restituée dans la sous-région de la carte anatomique.

3. Système selon la revendication 2, dans lequel l'ensemble de circuits de traitement est configuré pour répéter automatiquement la restitution de la partie limitée dans le temps de la propagation de l'onde d'activation cardiaque sur la sous-région de la carte anatomique.

4. Système selon la revendication 1, dans lequel l'ensemble de circuits de traitement est configuré pour restituer la sous-région de la carte anatomique à partir d'un point de vue à l'intérieur de la carte anatomique.

5. Système selon la revendication 4, dans lequel l'ensemble de circuits de traitement est configuré pour restituer la sous-région de la carte anatomique à partir d'un point de vue à l'intérieur de la carte anatomique, alors que le point de vue est statique pendant la restitution de la partie limitée dans le temps de la propagation de l'onde d'activation cardiaque sur la sous-région de la carte anatomique.

6. Système selon la revendication 4, comprenant en outre une interface utilisateur pour recevoir une entrée utilisateur d'une manipulation d'une caméra virtuelle pour changer une vue restituée de la carte anatomique à partir de l'intérieur de la carte anatomique, dans lequel l'ensemble de circuits de traitement est configuré pour restituer la sous-région de la carte anatomique en réponse à l'entrée utilisateur.

7. Système selon la revendication 1, dans lequel l'ensemble de circuits de traitement est configuré pour restituer la sous-région de la carte anatomique à partir d'un point de vue extérieur à la carte anatomique.

8. Système selon la revendication 7, comprenant en outre une interface utilisateur pour recevoir une entrée utilisateur d'une sélection de la sous-région de la carte anatomique, dans lequel l'ensemble de circuits de traitement est configuré pour restituer la sous-région de la carte anatomique en réponse à l'entrée utilisateur.

9. Système selon la revendication 1, comprenant en outre une interface utilisateur pour recevoir une entrée utilisateur d'une vitesse de la restitution de la partie limitée dans le temps de la propagation de l'onde d'activation cardiaque, dans lequel l'ensemble de circuits de traitement est configuré pour restituer sur le dispositif d'affichage la partie limitée dans le temps de la propagation de l'onde d'activation cardiaque sur la sous-région de la carte anatomique en réponse à l'entrée utilisateur de la vitesse.

10. Système selon la revendication 1, comprenant en outre une interface utilisateur pour recevoir une entrée utilisateur d'une largeur de l'onde d'activation cardiaque, dans lequel l'ensemble de circuits de traitement est configuré pour restituer sur le dispositif d'affichage la partie limitée dans le temps de la propagation de l'onde d'activation cardiaque sur la sous-région de la carte anatomique en réponse à l'entrée utilisateur de la largeur de l'onde d'activation cardiaque.

11. Procédé médical d'amélioration de commande de propagation d'onde, le procédé comprenant :
l'identification de temps d'activation locale (LAT) à partir d'une activité électrique de tissu d'une cavité d'un coeur (26) captée par des électrodes (52, 55) d'un cathéter (40) inséré dans la cavité et l'association des temps d'activation locale à des positions respectives sur la surface d'une carte anatomique (62) de la cavité du coeur ;
le calcul d'une propagation d'une onde d'activation cardiaque (60) sur la carte anatomique de la cavité du coeur à partir d'un moment de début d'un cycle cardiaque jusqu'à un moment de fin du cycle cardiaque en réponse à l'activité électrique captée, dans lequel la propagation de l'onde d'activation cardiaque est calculée à l'aide d'une fenêtre glissante des temps d'activation locale ;
la restitution d'une sous-région (64) de la carte anatomique sur un dispositif d'affichage (27) ;
la sélection d'une partie limitée dans le temps de la propagation de l'onde d'activation cardiaque commençant à un moment postérieur au moment de début, dans lequel le moment postérieur au moment de début est le moment le plus tôt où l'onde arriverait dans la sous-région de la carte anatomique ; et
la restitution de la partie limitée dans le temps de la propagation de l'onde d'activation cardiaque sur la sous-région de la carte anatomique sur le dispositif d'affichage.

12. Procédé selon la revendication 11, dans lequel la sélection comporte la sélection de la partie limitée dans le temps de la propagation de l'onde d'activation cardiaque se terminant à un moment antérieur au moment de fin, en réponse au moment où la propagation de l'onde d'activation cardiaque s'achèverait pour être restituée dans la sous-région de la carte anatomique.

13. Procédé selon la revendication 12, comprenant en outre la répétition automatique de la restitution de la partie limitée dans le temps de la propagation de l'onde d'activation cardiaque sur la sous-région de la carte anatomique.

14. Procédé selon la revendication 11, dans lequel la restitution de la sous-région comporte la restitution de la sous-région de la carte anatomique à partir d'un point de vue à l'intérieur de la carte anatomique.

15. Procédé selon la revendication 14, dans lequel la restitution de la sous-région comporte la restitution de la sous-région de la carte anatomique à partir d'un point de vue à l'intérieur de la carte anatomique, alors que le point de vue est statique pendant la restitution de la partie limitée dans le temps de la propagation de l'onde d'activation cardiaque sur la sous-région de la carte anatomique.

16. Procédé selon la revendication 14, comprenant en outre la réception d'une entrée utilisateur d'une manipulation d'une caméra virtuelle pour changer une vue restituée de la carte anatomique à partir de l'intérieur de la carte anatomique, dans lequel la restitution de la sous-région comporte la restitution de la sous-région de la carte anatomique en réponse à l'entrée utilisateur.

17. Procédé selon la revendication 11, dans lequel la restitution de la sous-région comporte la restitution de la sous-région de la carte anatomique à partir d'un point de vue extérieur à la carte anatomique.

18. Procédé selon la revendication 17, comprenant en outre la réception d'une entrée utilisateur d'une sélection de la sous-région de la carte anatomique, dans lequel la restitution de la sous-région comporte la restitution de la sous-région de la carte anatomique en réponse à l'entrée utilisateur.

19. Procédé selon la revendication 11, comprenant en outre la réception d'une entrée utilisateur d'une vitesse de la restitution de la partie limitée dans le temps de la propagation de l'onde d'activation cardiaque, dans lequel la restitution de la sous-région comporte la restitution de la partie limitée dans le temps de la propagation de l'onde d'activation cardiaque sur la sous-région de la carte anatomique en réponse à l'entrée utilisateur de la vitesse.

20. Procédé selon la revendication 11, comprenant en outre une interface utilisateur pour recevoir une entrée utilisateur d'une largeur de l'onde d'activation cardiaque, dans lequel l'ensemble de circuits de traitement est configuré pour restituer sur le dispositif d'affichage la partie limitée dans le temps de la propagation de l'onde d'activation cardiaque sur la sous-région de la carte anatomique en réponse à l'entrée utilisateur de la largeur de l'onde d'activation cardiaque.

21. Produit logiciel, comprenant un support lisible par ordinateur non transitoire dans lequel sont stockées des instructions de programme, lesquelles instructions, lorsqu'elles sont lues par une unité centrale de traitement (CPU) (41), amènent la CPU à :
identifier des temps d'activation locale (LAT) à partir d'une activité électrique de tissu d'une cavité d'un coeur (26) captée par des électrodes (52, 55) d'un cathéter (40) inséré dans la cavité et associer les LAT à des positions respectives sur la surface d'une carte anatomique (62) de la cavité du coeur ;
calculer une propagation d'une onde d'activation cardiaque (60) sur la carte anatomique de la cavité du coeur à partir d'un moment de début d'un cycle cardiaque jusqu'à un moment de fin du cycle cardiaque en réponse à l'activité électrique captée, dans lequel la propagation de l'onde d'activation cardiaque est calculée à l'aide d'une fenêtre glissante des temps d'activation locale ;
restituer une sous-région (64) de la carte anatomique sur un dispositif d'affichage (27) ;
sélectionner une partie limitée dans le temps de la propagation de l'onde d'activation cardiaque commençant à un moment postérieur au moment de début, dans lequel le moment postérieur au moment de début est le moment le plus tôt où l'onde arriverait dans la sous-région de la carte anatomique ; et
restituer la partie limitée dans le temps de la propagation de l'onde d'activation cardiaque sur la sous-région de la carte anatomique sur le dispositif d'affichage.
